# EUROPÄISCHE PATENTSCHRIFT

(11) **EP 3 020 346 B1**
(45) Veröffentlichungstag und Bekanntmachung des Hinweises auf die Patenterteilung: **26.10.2016**
(21) Anmeldenummer: 15003125.0
(22) Anmeldetag: 31.10.2015
(51) Int. Cl.: A61B 17/29

(54) **MEDIZINISCHES INSTRUMENT**
MEDICAL INSTRUMENT
DISPOSITIF MÉDICAL

(30) Priorität: 14.11.2014 DE 102014016790
(43) Veröffentlichungstag der Anmeldung: 18.05.2016
(73) Patentinhaber: Karl Storz GmbH & Co. KG, 78532 Tuttlingen (DE)
(72) Erfinder: Volkmer, Dominik, 78567 Fridingen (DE); Stefan, Jochen, 88639 Wald (DE)
(74) Vertreter: Hofmeister, Frank

(56) Entgegenhaltungen:
- WO-A1-2011/156168
- WO-A1-2013/154158
- WO-A1-2013/154700
- US-A- 5 607 449
- US-A1- 2010 331 857
- US-A1- 2012 310 222

## Beschreibung

Die Erfindung betrifft ein medizinisches Instrument mit einem hohlen Schaft, an dessen proximalem Ende eine Handhabe angeordnet ist und an dessen distalem Ende eine Werkzeugspitze angeordnet ist, wobei die Werkzeugspitze ein Werkzeug aufweist, das über ein federbelastetes Zugseil betätigbar ist und wobei die Werkzeugspitze um die Längsachse des Schaftes rotierbar sowie gegenüber der Längsachse des Schaftes abwinkelbar am Schaft gelagert ist, wobei das Betätigen des Zugseils sowie das Rotieren und Abwinkeln der Werkzeugspitze über jeweils separate Antriebe erfolgt, die an der Handhabe angeordnet sind.

Um dem Operateur bei der Verwendung eines medizinischen Halte- und/oder Greifinstruments ein hohes Maß an Bewegungsfreiheit zu geben, ist es aus der Praxis bekannt, zusätzlich zum bloßen Betätigen eines distalseitigen Werkzeugs durch Öffnen und Schließen der Maulteile die Werkzeugspitze des medizinischen Instruments so auszugestalten, dass diese um die Längsachse des Instruments rotierbar sowie gegenüber der Längsachse des Instruments verschwenkbar ausgebildet sein kann.

Ein gattungsgemäßes medizinisches Instrument ist beispielsweise aus der WO 2013/154158 A1 bekannt. Bei diesem bekannten medizinischen Instrument besteht aufgrund von auftretenden Reibungskräften die Gefahr, dass das Zugseil beim Rotieren der Werkzeugspitze auf Torsion beansprucht wird, wodurch die Standzeit des Zugseils, insbesondere bei wechselnder Drehrichtung der Rotation der Werkzeugspitze, beeinträchtigt werden kann.

Davon ausgehend liegt der Erfindung die **Aufgabe** zugrunde, ein medizinisches Instrument der eingangs genannten Art so auszugestalten, dass eine torsionsfreie Lagerung des Zugseils gewährleistet ist.

Die Lösung dieser Aufgabenstellung ist erfindungsgemäß dadurch gekennzeichnet, dass an dem in der um die Längsachse des Schaftes rotierbaren Werkzeugspitze angeordneten distalen Ende des Zugseils eine das Zugseil gegen Torsion schützende Verdrehsicherung angeordnet ist, wobei die Verdrehsicherung zumindest teilweise am nicht rotierenden Schaft gelagert ist.

Durch die Verwendung der erfindungsgemäßen Verdrehsicherung, die zumindest teilweise am nicht rotierenden Schaft, also am starren Teil des Instruments gelagert ist, ist es erstmalig zuverlässig möglich, eine Torsionsbelastung des Zugseils zu verhindern.

Gemäß einer praktischen Ausführungsform der Erfindung wird vorgeschlagen, dass die Verdrehsicherung zweiteilig aufgebaut ist und aus einem mit dem Zugseil verbundenen ersten Teil und einem am nicht rotierbaren Schaft gelagerten zweiten Teil besteht, wobei beide Teile der Verdrehsicherung derart in Wirkverbindung miteinander stehen, dass der zweite Teil eine Verdrehung des ersten Teil relativ zum zweiten Teil blockiert.

Zur Ausbildung der beiden Teile der Verdrehsicherung wird mit der Erfindung vorgeschlagen, dass der mit dem Zugseil verbundene erste Teil der Verdrehsicherung als das Zugseil koaxial umschließendes Rohr ausgebildet ist und, dass der am Schaft gelagerte zweite Teil der Verdrehsicherung als das Rohr zumindest abschnittweise umgreifende Hülse ausgebildet ist.

Um zu gewährleisten, dass das Zugseil in axialer Richtung verlagerbar ist, wie dies zur Betätigung des distalseitigen Werkzeugs erforderlich ist, wird mit der Erfindung weiterhin vorgeschlagen, dass die beiden Teile der Verdrehsicherung in Richtung der Längsachse des Schaftes relativ zueinander verschiebbar sind.

Gemäß einer ersten Ausführungsform zur Ausbildung der zweiteiligen Verdrehsicherung wird erfindungsgemäß vorgeschlagen, dass das den ersten Teil der Verdrehsicherung bildende Rohr eine im Querschnitt unrunde Außenkontur aufweist ist und, dass die den zweiten Teil der Verdrehsicherung bildende Hülse in dem das Rohr umgreifenden Bereich eine innere Oberflächenkontur aufweist, die eine korrespondierende Gegenkontur zur Außenkontur des Rohres in diesem umgriffenen Bereich bildet.

Durch diese zumindest in Teilbereichen formschlüssige Anlage der beiden Bauteile Rohr einerseits und Hülse andererseits aneinander wird eine mögliche Rotation des Rohres um die Längsachse des Schaftes innerhalb der Hülse verhindert.

Die das Verdrehen des Rohres relativ zur Hülse verhindernde unrunde Außenkontur des Rohres sowie die korrespondierende Gegenkontur der Hülse wird erfindungsgemäß dadurch erzielt, dass das den ersten Teil der Verdrehsicherung bildende Rohr auf seiner äußeren Mantelfläche zwei in Richtung der Längsachse des Schaftes verlaufende Abflachungen aufweist und, dass die den zweiten Teil der Verdrehsicherung bildende Hülse in dem das Rohr umgreifenden Bereich an der dem Rohr zugewandten Innenseite zwei korrespondierende Abflachungen ausgebildet sind, wobei die auf der äußeren Mantelfläche des Rohres ausgebildeten Abflachungen vorteilhafterweise um 180° versetzt zueinander angeordnet sind.

Durch das gegenseitige aneinander Anliegen der planen Abflachungen des Rohres und der planen Abflachungen der Hülse wird ein Verdrehen des in dem Rohr festgelegten Zugseils beim Rotieren der Werkzeugspitze um die Längsachse des Schaftes zuverlässig und dauerhaft unterbunden.

Schließlich wird mit einer zweiten Ausführungsform zur Ausbildung der zweiteiligen Verdrehsicherung vorgeschlagen, dass in dem den ersten Teil der Verdrehsicherung bildende Rohr ein in Richtung der Längsachse des Schaftes verlaufendes Langloch ausgebildet ist und, dass in der den zweiten Teil der Verdrehsicherung bildenden Hülse ein radial verlaufender und in das Langloch des Rohres hineinragender Stift angeordnet ist.

Weitere Merkmale und Vorteile der Erfindung ergeben sich anhand der zugehörigen Zeichnungen, in denen zwei Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine perspektivische Seitenansicht eines erfindungsgemäßen medizinischen Instruments;

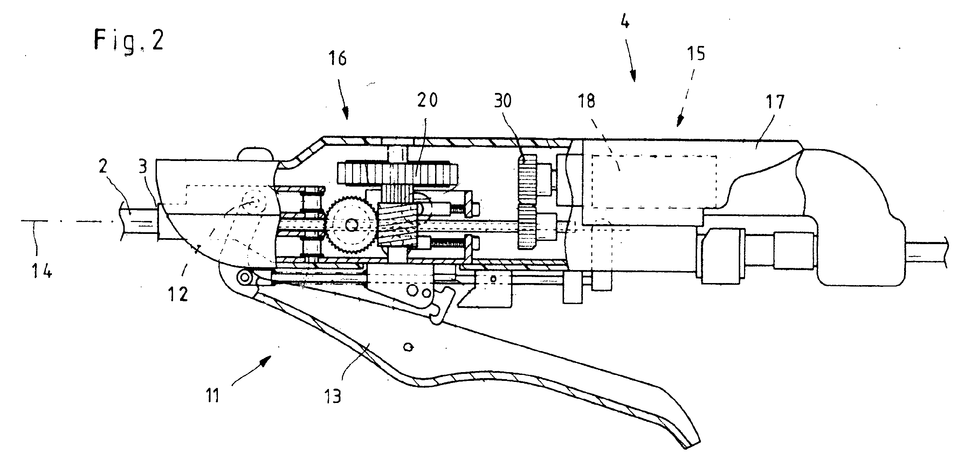

- Fig. 2: eine teilweise geschnittene Seitenansicht der Handhabe des medizinischen Instruments gemäß Fig. 1;
- Fig. 3: eine perspektivische Seitenansicht des Details III gemäß Fig. 1, eine erste Ausführungsform der Verdrehsicherung darstellend;
- Fig. 4: eine Explosionszeichnung der Darstellung gemäß Fig. 3;
- Fig. 5: einen Längsschnitt durch die Darstellung gemäß Fig. 3;
- Fig. 6: eine freigeschnittene vergrößerte Detailansicht der Verdrehsicherung gemäß Fig. 3 und
- Fig. 7: eine Ansicht gemäß Fig. 6, jedoch eine zweite Ausführungsform der Verdrehsicherung darstellend.

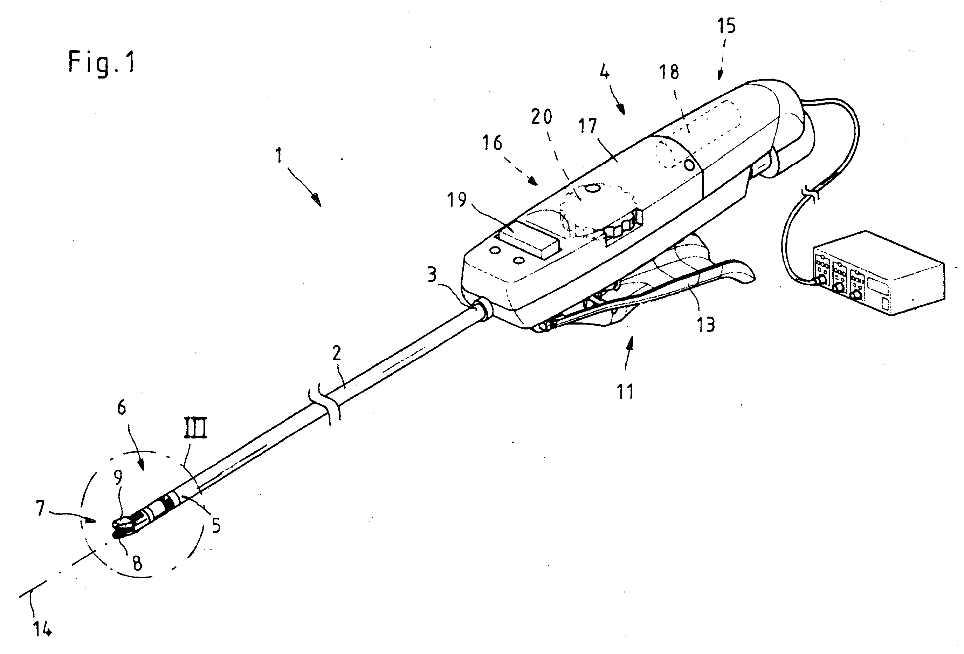

Fig. 1 zeigt eine perspektivische Seitenansicht eines medizinischen Instruments 1, das im Wesentlichen aus einem hohlen Schaft 2, einer am proximalem Ende 3 des Schaftes 2 angeordneten Handhabe 4 sowie einer am distalem Ende 5 des Schaftes 2 angeordneten Werkzeugspitze 6 besteht.

Die Werkzeugspitze 6 ihrerseits weist an ihrem distalen Ende ein Werkzeug 7 auf, das bei der dargestellten Ausführungsform aus einem starren Maulteil 8 sowie einem gegenüber dem starren Maulteil 8 verschwenkbaren Maulteil 9 besteht.

Alternativ zu der dargestellten Ausführung des Werkzeugs 7 als aus zwei Maulteile 8 und 9 bestehendes Greif- und Haltewerkzeug kann das Werkzeug beispielsweise auch als Schneidwerkzeug ausgebildet sein. Ebenso besteht die Möglichkeit, beide Maulteile des Werkzeugs 7 als verschwenkbare Maulteile 9 auszubilden.

Die Maulteile 8 und 9 des Werkzeugs 7 sind zwischen einer offenen Ausgangsstellung sowie einer geschlossenen Haltestellung verstellbar. Die Betätigung des Werkzeugs 7, im dargestellten Ausführungsbeispiel also das Verschwenken des Maulteils 9 erfolgt über ein im hohlen Schaft 2 gelagertes Zugseil 10, das mit einem an der Handhabe 4 angeordneten Antrieb 11 in Wirkverbindung steht. Der Antrieb 11 zur Betätigung des Werkzeugs 7 ist als um eine Schwenkachse 12 verschwenkbar an der Handhabe 4 gelagerter Griff 13 ausgebildet.

Zusätzlich zum Betätigen des Werkzeugs 7 über den Antrieb 11 ist die Werkzeugspitze 6 um die Längsachse 14 des Schaftes 2 rotierbar sowie gegenüber der Längsachse 14 des Schaftes 2 abwinkelbar am distalen Ende 5 des Schaftes 2 gelagert. Das Rotieren und Abwinkeln der Werkzeugspitze 6 erfolgt über jeweils separate, an der Handhabe 4 angeordnete Antriebe 15 und 16.

Alternativ zum dargestellten Verschwenken der Werkzeugspitze 6 relativ zur Längsachse 14 des Schaftes 2 nach rechts und links ist es ebenso möglich, das medizinische Instrument 1 so auszugestalten, dass die Werkzeugspitze 6 relativ zur Längsachse 14 des Schaftes 2 nach oben und unten verschwenkbar ist.

Der Antrieb 15 zum Rotieren der Werkzeugspitze 6 um die Längsachse 14 des Schaftes 2 ist als im Handhabengehäuse 17 angeordneter elektrischer oder pneumatischer Motor 18 ausgebildet, der über einen am distalen Ende der Handhabe 4 angeordneten Kippschalter 19 aktivierbar ist, um die Werkzeugspitze 6 entweder im Uhrzeigersinn oder aber im Gegenuhrzeigersinn um die Längsachse 14 des Schaftes 2 rotieren zu lassen.

Der Antrieb 16 zum Verschwenken der Werkzeugspitze 6 relativ zur Längsachse 14 des Schaftes 2 ist als die Handhabe 4 beidseitig seitlich durchragendes Drehrad 20 ausgebildet, bei dessen Verdrehen die Werkzeugspitze 6 entsprechend der Drehrichtung des Drehrads 20 relativ zur Längsachse 14 des Schaftes 2 verschwenkt wird.

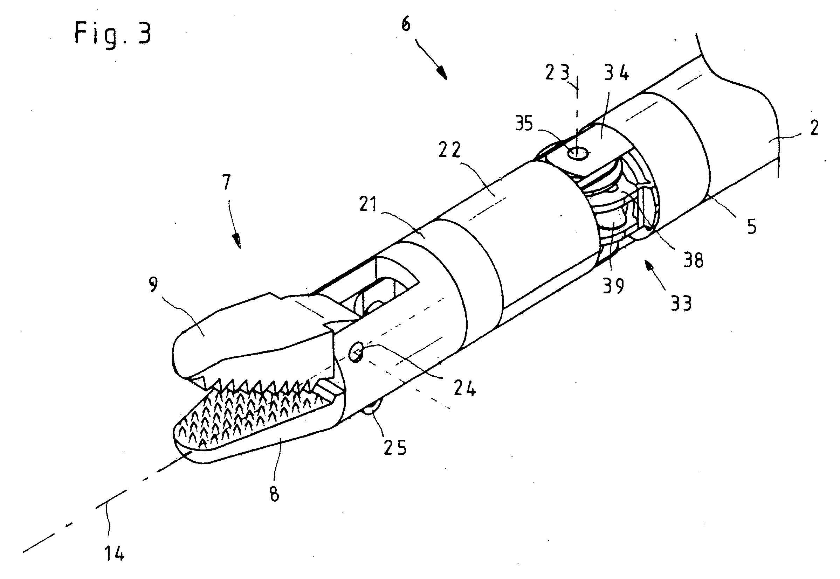

Die Abbildungen Fig. 3 bis Fig. 5 zeigen eine perspektivische Ansicht des distalen Endes 5 des Schaftes 2 und der mit dem Werkzeug 7 versehenen Werkzeugspitze 6, eine Explosionszeichnung dieses Instrumentenabschnitts sowie einen Längsschnitt durch diesen Instrumentenabschnitt.

Wie aus den Darstellungen gemäß Fig. 3 bis 5 ersichtlich, umfasst die Werkzeugspitze 6 das Werkzeug 7 mit den zu öffnenden und zu schließenden Maulteilen 8 und 9, eine um die Längsachse 14 des Schaftes 2 rotierbare Hülse 21, die fest mit dem starren Maulteil 8 des Werkzeugs 7 verbunden ist, sowie eine Dreheinheit 22, über die die Werkzeugspitze 6 um die Schwenkachse 23 relativ zur Längsachse 14 des Schaftes 2 verschwenkbar ist.

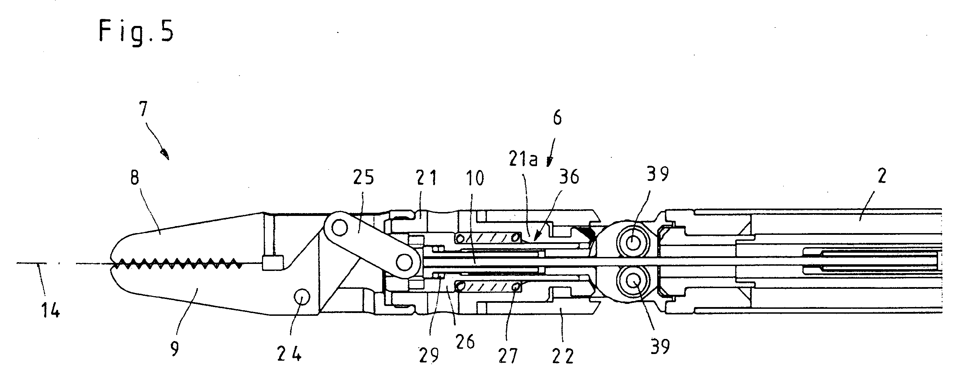

Wie aus Fig. 5 ersichtlich, sind das starre Maulteil 8 und das verschwekbare Maulteil 9 über einen Stift 24 miteinander verbunden, der gleichzeitig die Schwenkachse bildet, um die das verschwenkbare Maulteil 9 gegenüber dem starren Maulteit 8 zum Öffnen und Schließen des Werkzeugs 7 verschwenkbar ist. Das Betätigen des verschwenkbaren Maulteils 9 durch das Zugseil 10 erfolgt bei der dargestellten Ausführungsform indirekt über einen Anlenkhebel 25, der mit einem Ende verschwenkbar am verschwenkbaren Maulteil 9 gelagert ist und der mit seinem anderen Ende verschwenkbar an einem Übertragungselement 26 gelagert ist, das zur lagernden Aufnahme des distalen Endes des Zugseils 10 dient.

Das Übertragungselement 26 ist, wie aus Fig. 5 ersichtlich, als in der Hülse 21 der Werkzeugspitze 6 gelagertes hohles Rohr ausgebildet, das distalseitig einen den Außendurchmesser des Rohres radial erweiternden Kopf 26a aufweist. Das das Übertragungselement 26 bildende hohle Rohr ist in Richtung der Längsachse 14 des Schaftes 2 axialverschiebbar in der Hülse 21 gelagert. Zwischen der Hülse 21 und dem Übertragungselement 26 ist eine Federelement 27 in Form einer Druckfeder gelagert, das sich distalseitig am Kopf 26a des Übertragungselements 26 abstützt und proximalseitig an einem den Innendurchmesser der Hülse 21 verringernden Absatz 21a anliegt, wodurch das Übertragungselement 26 durch das Federelement 27 in die distale Richtung vorgespannt ist.

Im Inneren des hohlen Übertragungselements 26 ist ein weiteres hohles Rohr 28 angeordnet, in dem das distale Ende des Zugseils 10, beispielsweise durch Verlöten, fixiert ist. Das zur Aufnahme des Zugseils 10 dienende Rohr 28 weist distalseitig einen den Außendurchmesser des Rohres 28 radial erweiternden Kopf 28a auf, über den das Rohr 28 auf der Innenseite des Übertragungselements 26 am Kopf 26a des Übertragungselements 26 anliegt, wobei zwischen dem Kopf 28a des mit dem Zugseil 10 verbundenen Rohres 28 und dem Kopf 26a des Übertragungselements 26 vorteilhafterweise ein Drehlager 29, vorzugsweise aus dem Werkstoff PEEK (Polyetheretherketone), angeordnet ist.

Mit diesem zuvor beschriebenen Aufbau lässt sich die Betätigung des verschwenkbaren Maulteils 9 des Werkzeugs 7 wie folgt beschreiben:
Distalseitig steht das zur Betätigung des verschwenkbaren Maulteils 9 dienende Zugseil 10 über das Rohr 28, das Übertragungselement 26 und den Anlenkhebel 25 mit dem verschwenkbaren Maulteil 9 des Werkzeugs 7 in Wirkverbindung. Proximalseitig steht das Zugseil 10 in Wirkverbindung mit dem verschwenkbaren Griff 13 der Handhabe 4.

In der unbelasteten Ausgangsposition drückt das koaxial auf dem Rohr 28 angeordnete Federelement 27 das Übertragungselement 26 nach distal, wodurch über den Anlenkhebel 25 das verschwenkbare Maulteil 9 des Werkzeugs 7 in die offene Ausgangsstellung überführt wird. Die Federbelastung des Übertragungselements 26 nach distal bewirkt gleichzeitig auch, dass das fest mit dem Zugseil 10 verbundene Rohre 28 und somit auch das Zugseil 10 nach distal vorgespannt ist. In dieser Position des Zugseils 10 befindet sich auch der um die Schwenkachse 12 verschwenkbare Griff 13 in der Ausgangsposition, in der dieser in radialer Richtung weit von der Handhabe 4 beabstandet ist.

Wenn der Bediener des medizinischen Instruments 1 den Griff 13 ergreift und zur Handhabe 4 hin drückt, bewirkt diese Verlagerung des Griffes 13 über einen nicht näher beschriebenen Übertragungsmechanismus eine Zugkraft auf das Zugseil 10 in die proximale Richtung.

Diese Zugbelastung des Zugseils 10 in die proximale Richtung bewirkt, dass über den Kopf 28a des Rohres 28, in dem das Zugseil 10 fixiert ist, das Übertragungselement 26 gegen die Kraft des Federelements 27 nach proximal gezogen wird, wodurch auch der mit dem Übertragungselement 26 verbundene Anlenkhebel 25 nach proximal verlagert wird. Diese Verlagerung des Anlenkhebels 25 nach proximal bewirkt das Überführen des verschwenkbaren Maulteils 9 des Werkzeugs 7 in die geschlossene Arbeitsstellung.

Sobald der Bediener des medizinischen Instruments 1 den Griff 13 der Handhabe 4 wieder frei gibt, drückt das Federelement 27 sowohl das verschwenkbare Maulteil 9 als auch den Griff 13 wieder in die eingangs genannte Ausgangsposition, in der das Werkzeug 7 geöffnet ist.

Zusätzlich zum Öffnen und Schließen der Maulteile 8 und 9 des Werkzeugs 7 am distalen Ende der Werkzeugspitze 6 ist die Werkzeugspitze 6 um die Längsachse 14 des Schaftes 2 rotierbar sowie gegenüber der Längsachse 14 des Schaftes 2 abwinkelbar am distalen Ende 5 des Schaftes 2 gelagert ist.

Der Aufbau dieser Dreh- und Schwenkmechanismen ist insbesondere den Abbildungen Fig. 3 bis Fig. 5 zu entnehmen.

Die Rotation der Werkzeugspitze 6 um die Längsachse 14 des Schaftes 2 wird angetrieben über den in der Handhabe 4 angeordneten elektrisch oder pneumatisch betriebenen Motor 18, der über anflanschbare äußere Anschlüsse aktivierbar und über den Kippschalter 19 ein- und ausschaltbar ist.

Die vom Motor 18 erzeugte Drehbewegung wird auf ein im Handhabengehäuse 17 angeordnetes Zahnradgetriebe 30 übertragen, das eine im Schaft 2 angeordnete Hülse 31 so antreibt, dass diese um die Längsachse 14 des Schaftes 2 rotiert. Am distalen Ende der Hülse 31, welches am distalen Ende 5 des Schaftes 2 gelegen ist, ist an der Hülse 31 eine Kegelradverzahnung 31 a ausgebildet.

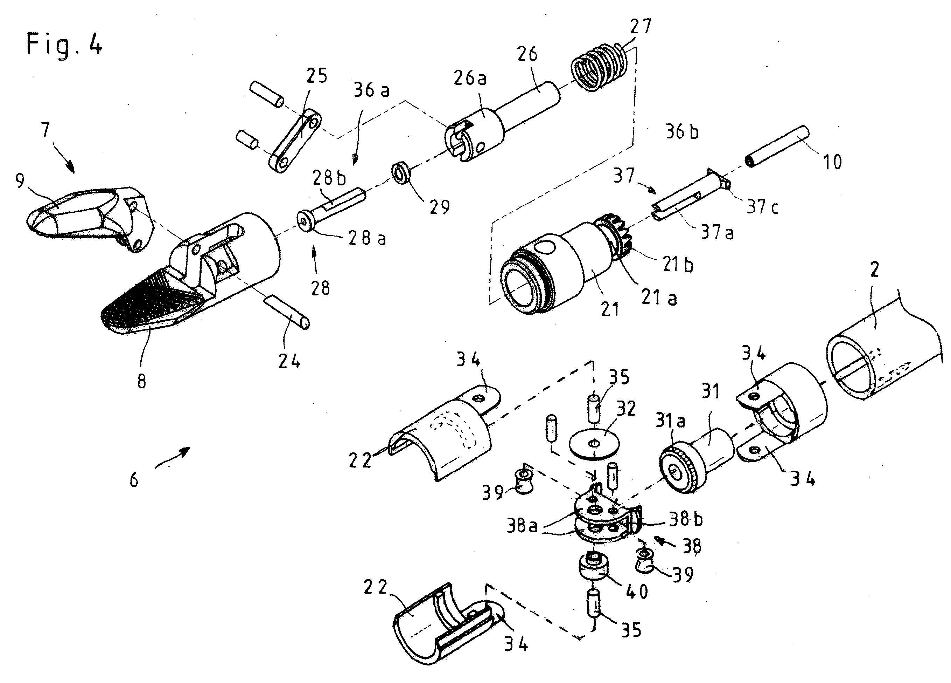

Wie aus Fig. 4 ersichtlich, kämmt die Kegelradverzahnung 31a der Hülse 31 mit einem Kegelzahnrad 32, das die Verbindung zwischen dem distalen Ende 5 des Schaftes 2 und der Werkzeugspitze 6 bildet.

Wie aus den Abbildungen Fig. 3 und 5 zu erkennen ist, ist zwischen dem distalen Ende 5 des Schaftes 2 und der Werkzeugspitze 6 ein Spalt 33 ausgebildet, der bei der dargestellten Ausführungsform des medizinischen Instruments 1 von jeweils zwei Laschen 34 überbrückt wird, die vom radial äußeren Rand des distalen Endes 5 des Schaftes 2 in Richtung der Längsachse 14 des Schaftes 2 ausgerichtet nach distal in Richtung der Werkzeugspitze 6 vorstehen sowie vom radial äußeren Rand des proximalen Endes der Dreheinheit 22 in Richtung der Längsachse 14 des Schaftes 2 ausgerichtet nach proximal in Richtung des Schaftes 2 vorstehen. Diese jeweils zwei Laschen des Schaftes 2 und der Dreheinheit 22 sind um 180° versetzt zueinander angeordnet und im Überlappungsbereich über Bolzen 35 miteinander verbunden.

Einer dieser Bolzen 35 bildet gleichzeitig die Lagerachse für das Kegelzahnrad 32. Auf Seiten der Werkzeugspitze 6 kämmt das Kegelzahnrad 32 mit einer Kegelradverzahnung 21b, die am proximalen Ende der Hülse 21 ausgebildet ist.

Mit diesem zuvor beschriebenen Aufbau lässt sich der Betrieb der Rotation der Werkzeugspitze 6 um die Längsachse 14 des Schaftes 2 wie folgt beschreiben:
Sobald der Bediener des medizinischen Instruments 1 den Kippschalter 19 betätigt, wird der Motor 18 eingeschaltet und beginnt eine Drehbewegung zu erzeugen. Diese Drehbewegung wird über das Zahnradgetriebe 30 auf die im Inneren des Schaftes 2 angeordnete Hülse 31 übertragen, so dass die Hülse 31 um die Längsachse 14 des Schaftes 2 rotiert. Die Rotation der Hülse 31 wird über die distalseitige Kegelradverzahnung 31a der Hülse 31 auf das Kegelzahnrad 32 und von dort weiter über die proximalseitige Kegelradverzahnung 21 b der Hülse 21 auf die an der Werkzeugspitze 6 angeordnete Hülse 21 so übertragen, dass die Hülse 21 um die Längsachse 14 des Schaftes 2 rotiert.

Aufgrund der festen Verbindung der Hülse 21 mit dem starren Maulteil 8 des Werkzeugs 7 rotiert auch das gesamte Werkzeug 7 um die Längsachse 14 des Schaftes 2.

Je nachdem, in welche Richtung der Kippschalter 19 betätigt wird, erfolgt die Rotation der Werkzeugspitze 6 um die Längsachse 14 des Schaftes 2 im Uhrzeigersinn oder im Gegenuhrzeigersinn. Eine gradmäßige Beschränkung der Rotationswinkels besteht bei der dargestellten Ausführungsform des medizinischen Instruments 1 nicht.

Auch während der Rotation der Werkzeugspitze 6 um die Längsachse 14 des Schaftes 2 ist es möglich, die Maulteile 8 und 9 des Werkzeugs 7 durch Betätigung des an der Handhabe 4 gelagerten Griffs 13 zu schließen und wieder zu öffnen.

Bei der Rotation der Werkzeugspitze 6 um die Längsachse 14 des Schaftes 2 besteht trotz der Verwendung des für die Lagerung des distalen Endes des Zugseils 10 im Übertragungselement 26 verwendeten Drehlagers 29 die Gefahr, dass das Zugseil 10 aufgrund von auftretenden Reibkräften auf Torsion beansprucht wird. Diese insbesondere in wechselnden Drehrichtungen auftretenden Torsionskräfte könnten zu einer deutlichen Herabsetzung der Lebensdauer des Zugseils 10 führen.

Um zu gewährleisten, dass das Zugseil 10 dauerhaft torsionsfrei in der rotierbaren Werkzeugspitze 6 gelagert ist, ist bei dem dargestellten medizinischen Instrument 1 eine Verdrehsicherung 36 vorgesehen, die jegliches Mitdrehen des Zugseils 10 mit der um die Längsachse 14 des Schaftes 2 rotierenden Werkzeugspitze 6 unterbindet.

Wie aus den Abbildungen Fig. 4, 6 und 7 ersichtlich, ist die Verdrehsicherung 36 bei der dargestellten Ausführungsform zweiteilig aufgebaut und besteht aus einem mit dem Zugseil 10 verbundenen ersten Teil 36a und einem am nicht rotierbaren Schaft 2 gelagerten zweiten Teil 36b.

Während der mit dem Zugseil 10 verbundene erste Teil 36a der Verdrehsicherung 36 durch das das Zugseil 10 distalseitig koaxial umschließende Rohr 28, in dem das Zugseil 10 fixiert ist gebildet wird, ist der am Schaft 2 gelagerte zweite Teil 36b der Verdrehsicherung 36 als das Rohr 28 zumindest abschnittweise umgreifende Hülse 37 ausgebildet.

Entscheidend für den Schutz des Zugseils 10 gegen Torsion ist, dass beide Teile 36a und 36b der Verdrehsicherung 36 derart in Wirkverbindung miteinander stehen, dass der am nicht rotierbaren Schaft 2 gelagerte zweite Teil 36b eine Verdrehung des mit dem Zugseil 10 verbundenen ersten Teil 36a relativ zum zweiten Teil 36b blockiert.

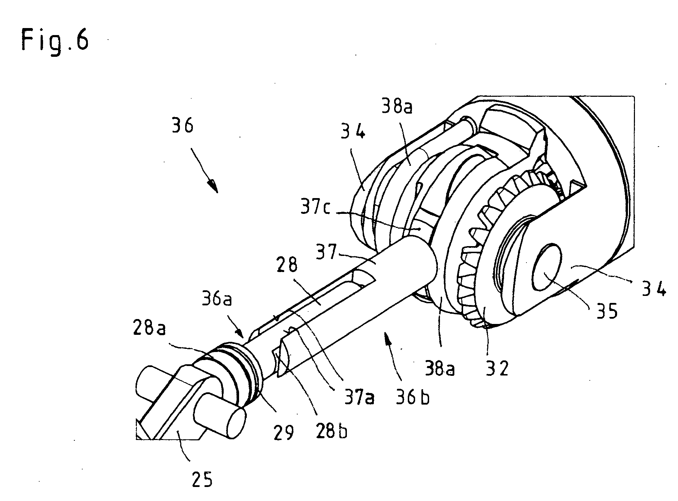

Wie insbesondere aus Fig. 6 ersichtlich, erfolgt dieses Blockieren einer möglichen Verdrehung des ersten Teils 36a der Verdrehsicherung 36 relativ zum zweiten Teil 36b der Verdrehsicherung 36 bei der in dieser Abbildung dargestellten ersten Ausführungsform der Verdrehsicherung 36 dadurch, dass das den ersten Teil 36a der Verdrehsicherung 36 bildende Rohr 28 eine im Querschnitt unrunde Außenkontur aufweist ist und, dass die den zweiten Teil 36b der Verdrehsicherung 36 bildende Hülse 37 in dem das Rohr 28 umgreifenden Bereich eine innere Oberflächenkontur aufweist, die eine korrespondierende Gegenkontur zur Außenkontur des Rohres 28 in diesem umgriffenen Bereich bildet. Durch diese zumindest in Teilbereichen formschlüssige Anlage der beiden Bauteile Rohr 28 einerseits und Hülse 37 andererseits aneinander wird eine mögliche Rotation des Rohres 28 um die Längsachse 14 des Schaftes 2 innerhalb der Hülse 37 verhindert.

Die das Verdrehen des Rohres 28 relativ zur Hülse 37 verhindernde unrunde Außenkontur des Rohres 28 sowie die korrespondierende Gegenkontur der Hülse 37 wird bei der dargestellten ersten Ausführungsform der Verdrehsicherung 36 dadurch erzielt, dass das den ersten Teil 36a der Verdrehsicherung 36 bildende Rohr 28 auf seiner äußeren Mantelfläche zwei in Richtung der Längsachse 14 des Schaftes 2 verlaufende Abflachungen 28b aufweist und, dass die den zweiten Teil 36b der Verdrehsicherung 36 bildende Hülse 37 in dem das Rohr 28 umgreifenden Bereich an der dem Rohr 28 zugewandten Innenseite zwei korrespondierende Abflachungen 37a ausgebildet sind. Die auf der äußeren Mantelfläche des Rohres 28 ausgebildeten Abflachungen 28b sind vorteilhafterweise um 180° versetzt zueinander angeordnet sind.

Durch das gegenseitige aneinander Anliegen der planen Abflachungen 28b des Rohres 28 und der planen Abflachungen 37a der Hülse 37 wird ein Verdrehen des in dem Rohr 28 festgelegten Zugseils 10 beim Rotieren der Werkzeugspitze 6 um die Längsachse 14 des Schaftes 2 zuverlässig und dauerhaft unterbunden.

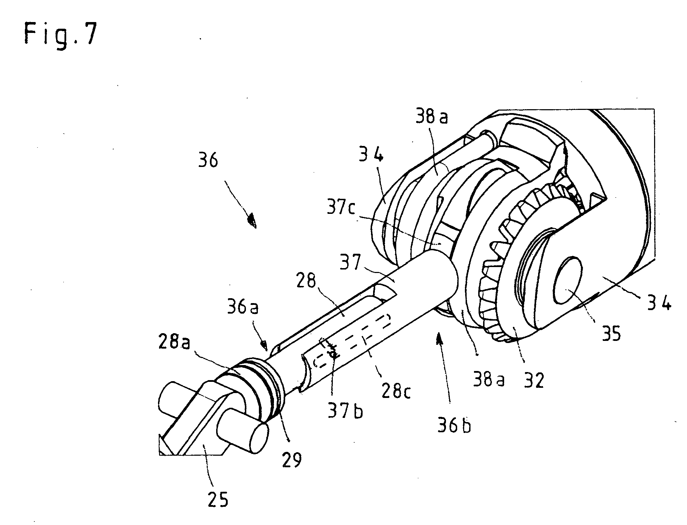

Gemäß der in der Abbildung Fig. 7 dargestellten zweiten Ausführungsform zur Ausbildung der Verdrehsicherung 36 ist in dem den ersten Teil 36a der Verdrehsicherung 36 bildende Rohr 28 ein in Richtung der Längsachse 14 des Schaftes 2 verlaufendes Langloch 28c ausgebildet, in das ein in der den zweiten Teil 36b der Verdrehsicherung 36 bildenden Hülse 37 angeordneter, in radialer Richtung verlaufender Stift 37b hineinragt.

Das Blockieren einer möglichen Verdrehung des ersten Teils 36a der Verdrehsicherung 36 relativ zum zweiten Teil 36b der Verdrehsicherung 36 erfolgt bei der in dieser Abbildung dargestellten zweiten Ausführungsform der Verdrehsicherung 36 dadurch, dass der Stift 37b, der im Inneren der mit dem nicht rotierbaren Schaft 2 verbundenen Hülse 37 angeordnet ist, infolge des Eintretens in das Langloch 28c, das im mit dem Zugseil 10 verbundenen Rohr 28 ausgebildet ist, eine mögliche Rotation des Rohres 28 um die Längsachse 14 des Schaftes 2 aufgrund der Rotation der Werkzeugspitze 6 blockiert.

Da das Zugseil 10 zum Öffnen und Schließen des Werkzeugs 7 eine Axialbewegung in Richtung der Längsachse 14 es Schaftes 2 ausführen können muss, ist es für die Ausbildung der Verdrehsicherung 36 zusätzlich unabdingbar, dass das Zugseil 10 axial verschiebbar innerhalb der Verdrehsicherung 36 angeordnet sein muss.

Wie aus den beiden in den Abbildungen Fig. 6 und 7 dargestellten Ausführungsformen zur Ausbildung der Verdrehsicherung 36 ersichtlich, sind beiden Teile 36a und 36b der Verdrehsicherung 36 in Richtung der Längsachse 14 des Schaftes 2 relativ zueinander verschiebbar ausgestaltet. Bei der in Fig. 6 dargestellten Ausführungsform erlauben die in Richtung der Längsachse 14 des Schaftes verlaufenden Abflachungen 28b des Rohres 28 und die ebenso verlaufenden Abflachungen 37a der Hülse 37 diese Axialbewegung der Bauteile Rohr 28 und Hülse 37 relativ zueinander.

Bei der in Fig. 7 dargestellten Ausführungsform ermöglicht das in Richtung der Längsachse 14 des Schaftes verlaufende Langloch 28c des Rohres 28 mit dem darin angeordneten Stift 37b der Hülse 37 diese Axialbewegung der Bauteile Rohr 28 und Hülse 37 relativ zueinander.

Um die Hülse 37 verdrehsicher am distalen Ende 5 des nicht rotierenden Schaftes 2 zu lagern, weist die Hülse 37 an ihrem proximalen Ende einen Sockel 37c auf, mit dem die Hülse in einem Lagerblock 38 gelagert ist. Wie aus den Abbildungen Fig. 3 und 4 ersichtlich, ist der Lagerblock im Spalt 33 zwischen dem distalen Ende 5 des Schaftes 2 und dem proximalen Ende der Werkzeugspitze 6 zwischen den den Spalt 33 überbrückenden Laschen 34 angeordnet, wobei die die Laschen 34 miteinander verbindenden Bolzen 35 endseitig im Lagerblock 38 gelagert sind. Die Schwenkachse 23, um die die Werkzeugspitze 6 gegenüber der Längsachse 14 des Schaftes 2 verschwenkbar ist, wird durch die Bolzen 35 gebildet.

Der Lagerblock 38 ist als in der Seitenansicht U-förmig so ausgebildet, dass sich die beiden parallelen Seiten 38a nach distal erstrecken und der die beiden Seiten 38a miteinander verbindende Steg 38b plan am distalen Ende 5 des Schaftes 2 anliegt. Die Seiten 38a des Lagerblocks 38 sind aus der Draufsicht halbkreisförmig ausgebildet.

Im Zwischenraum zwischen den beiden parallelen Seiten 38a des Lagerblocks 38 sind rechts und links der Längsachse 14 des Schaftes 2 zwei Umlenkrolle 39 angeordnet. Das Zugseil 10 erstreckt sich vom Schaft 2 kommend durch den Steg 38b des Lagerblocks 38 hin zur Werkzeugspitze 6.

Mit diesem zuvor beschriebenen Aufbau lässt sich der Betrieb des Verschwenkens der Werkzeugspitze 6 gegenüber der Längsachse 14 des Schaftes 2 wie folgt beschreiben:
Wenn der Bediener des medizinischen Instruments 1 das an der Handhabe 4 gelagerte Drehrad 20 betätigt, wird ein direkt mit dem Drehrad 20 in Wirkverbindung stehendes Übertragungselement in Form eines nicht dargestellten Seil- oder Kettenzugs in Bewegung gesetzt, das distalseitig mit einer drehbar am Lagerblock 38 gelagerten Antriebsrolle 40 in Wirkverbindung steht.

Über das als Seil- oder Kettenzug ausgebildete Übertragungselement wird die Drehbewegung des Drehrads 20 so auf die Antriebsrolle 40 übertragen, die verdrehfest mit einem der die Schwenkachse 23 bildenden Bolzen 35 verbunden ist, dass das Verdrehen der Antriebsrolle 40 das Verschwenken der Werkzeugspitze 6 gegenüber der Längsachse 14 des Schaftes 2 und um die Schwenkachse 23 bewirkt.

Die im Lagerblock 38 gelagerten Umlenkrolle 39 dienen dabei zur seitlichen Führung des Zugseils 10. Der das proximale Ende der Hülse 37 bildende Sockel 37c ist so zwischen den Seiten 38a des Lagerblocks 38 gelagert, dass er die Schwenkbewegung um die Schwenkachse 23 mitmachen kann und zwischen den Seiten 38a des Lagerblocks 38 hin- und hergleitet.

Auch während des Verschwenkens der Werkzeugspitze 6 gegenüber der Längsachse 14 des Schaftes 2 ist es möglich, die Maulteile 8 und 9 des Werkzeugs 7 durch Betätigung des an der Handhabe 4 gelagerten Griffs 13 zu schließen und wieder zu öffnen.

Ein wie zuvor beschriebenes medizinisches Instrument 1 zeichnet sich dadurch aus, dass das Zugseil 10 bei der Rotation der Werkzeugspitze 6 um die Längsachse 14 des Schaftes 2 zuverlässig gegen Torsion geschützt ist.

**Bezugszeichenliste**

| | | | |
|---|---|---|---|
| 1 | medizinisches Instrument | 18 | Motor |
| 2 | Schaft | 19 | Kippschalter |
| 3 | proximales Ende | 20 | Drehrad |
| 4 | Handhabe | 21 | Hülse |
| 5 | distales Ende | 21a | Absatz |
| 6 | Werkzeugspitze | 21b | Kegelradverzahnung |
| 7 | Werkzeug | 22 | Dreheinheit |
| 8 | starres Maulteil | 23 | Schwenkachse |
| 9 | verschwenkbares Maulteil | 24 | Stift |
| 10 | Zugseil | 25 | Anlenkhebel |
| 11 | Antrieb (Werkzeug) | 26 | Übertragungselement |
| 12 | Schwenkachse | 26a | Kopf |
| 13 | Griff | 27 | Federelement |
| 14 | Längsachse | 28 | Rohr |
| 15 | Antrieb (rotieren) | 28a | Kopf |
| 16 | Antrieb (verschwenken) | 28b | Abflachung |
| 17 | Handhabengehäuse | 28c | Langloch |
| 29 | Drehlager | 39 | Umlenkrolle |
| 30 | Zahnradgetriebe | 40 | Antriebsrolle |
| 31 | Hülse | | |
| 31a | Kegelradverzahnung | | |
| 32 | Kegelzahnrad | | |
| 33 | Spalt | | |
| 34 | Lasche | | |
| 35 | Bolzen | | |
| 36 | Verdrehsicherung | | |
| 36a | erster Teil | | |
| 36b | zweiter Teil | | |
| 37 | Hülse | | |
| 37a | Abflachung | | |
| 37b | Stift | | |
| 37c | Sockel | | |
| 38 | Lagerblock | | |
| 38a | Seite | | |
| 38b | Steg | | |

## Patentansprüche

1. Medizinisches Instrument mit einem hohlen Schaft (2), an dessen proximalem Ende (3) eine Handhabe (4) angeordnet ist und an dessen distalem Ende (5) eine Werkzeugspitze (6) angeordnet ist, wobei die Werkzeugspitze (6) ein Werkzeug (7) aufweist, das über ein federbelastetes Zugseil (10) betätigbar ist und wobei die Werkzeugspitze (6) um die Längsachse (14) des Schaftes (2) rotierbar sowie gegenüber der Längsachse (14) des Schaftes (2) abwinkelbar am Schaft (2) gelagert ist, wobei das Betätigen des Zugseils (10) sowie das Rotieren und Abwinkeln der Werkzeugspitze (6) über jeweils separate Antriebe (11, 15, 16) erfolgt, die an der Handhabe (4) angeordnet sind,
**dadurch gekennzeichnet,**
**dass** an dem in der um die Längsachse (14) des Schaftes (2) rotierbaren Werkzeugspitze (6) angeordneten distalen Ende des Zugseils (10) eine das Zugseil (10) gegen Torsion schützende Verdrehsicherung (36) angeordnet ist, wobei die Verdrehsicherung (36) zumindest teilweise am nicht rotierenden Schaft (2) gelagert ist.

2. Medizinisches Instrument nach Anspruch 1, **dadurch gekennzeichnet, dass** die Verdrehsicherung (36) zweiteilig aufgebaut ist und aus einem mit dem Zugseil (10) verbundenen ersten Teil (36a) und einem am nicht rotierbaren Schaft (2) gelagerten zweiten Teil (36b) besteht, wobei beide Teile (36a und 36b) der Verdrehsicherung (36) derart in Wirkverbindung miteinander stehen, dass der zweite Teil (36b) eine Verdrehung des ersten Teil (36a) relativ zum zweiten Teil (36b) blockiert.

3. Medizinisches Instrument nach Anspruch 2, **dadurch gekennzeichnet, dass** der mit dem Zugseil (10) verbundene erste Teil (36a) der Verdrehsicherung (36) als das Zugseil (10) koaxial umschließendes Rohr (28) ausgebildet ist und, dass der am Schaft (2) gelagerte zweite Teil (36b) der Verdrehsicherung (36) als das Rohr (28) zumindest abschnittweise umgreifende Hülse (37) ausgebildet ist.

4. Medizinisches Instrument nach Anspruch 2 oder 3, **dadurch gekennzeichnet, dass** die beiden Teile (36a, 36b) der Verdrehsicherung (36) in Richtung der Längsachse (14) des Schaftes (2) relativ zueinander verschiebbar sind.

5. Medizinisches Instrument nach den Ansprüchen 3 und 4, **dadurch gekennzeichnet, dass** das den ersten Teil (36a) der Verdrehsicherung (36) bildende Rohr (28) eine im Querschnitt unrunde Außenkontur aufweist ist und, dass die den zweiten Teil (36b) der Verdrehsicherung (36) bildende Hülse (37) in dem das Rohr (28) umgreifenden Bereich eine innere Oberflächenkontur aufweist, die eine korrespondierende Gegenkontur zur Außenkontur des Rohres (28) in diesem umgriffenen Bereich bildet.

6. Medizinisches Instrument nach Anspruch 5, **dadurch gekennzeichnet, dass** das den ersten Teil (36a) der Verdrehsicherung (36) bildende Rohr (28) auf seiner äußeren Mantelfläche zwei in Richtung der Längsachse (14) des Schaftes (2) verlaufende Abflachungen (28b) aufweist und, dass die den zweiten Teil (36b) der Verdrehsicherung (36) bildende Hülse (37) in dem das Rohr (28) umgreifenden Bereich an der dem Rohr (28) zugewandten Innenseite zwei korrespondierende Abflachungen (37a) ausgebildet sind.

7. Medizinisches Instrument nach Anspruch 6, **dadurch gekennzeichnet, dass** die auf der äußeren Mantelfläche des Rohres (28) ausgebildeten Abflachungen (28b) um 180° versetzt zueinander angeordnet sind.

8. Medizinisches Instrument nach den Ansprüchen 3 und 4, **dadurch gekennzeichnet, dass** in dem den ersten Teil (36a) der Verdrehsicherung (36) bildende Rohr (28) ein in Richtung der Längsachse (14) des Schaftes (2) verlaufendes Langloch (28c) ausgebildet ist und, dass in der den zweiten Teil (36b) der Verdrehsicherung (36) bildenden Hülse (37) ein radial verlaufender und in das Langloch (28c) des Rohres (28) hineinragender Stift (37b) angeordnet ist.

## Claims

1. Medical instrument with a hollow shaft (2), at whose proximal end (3) a handle (4) is arranged and at whose distal end (5) a tool tip (6) is arranged, wherein the tool tip (6) has a tool (7) which can be activated by a spring-loaded pull cable (10) and wherein the tool tip (6) is mounted so that it can rotate about the lengthwise axis (14) of the shaft (2) and can bend on the shaft (2) relative to the lengthwise axis (14) of the shaft (2), wherein the activating of the pull cable (10) as well as the rotating and bending of the tool tip (6) occurs each time by separate drive units (11, 15, 16), which are arranged on the handle (4),
**characterized in that**
an anti-rotation device (36) protecting the pull cable (10) against torsion is arranged at the distal end of the pull cable (10) arranged in the tool tip (6) able to rotate about the lengthwise axis (14) of the shaft (2), while the anti-rotation device (36) is mounted at least partly on the non-rotating shaft (2).

2. Medical instrument according to claim 1, **characterized in that** the anti-rotation device (36) is two-part in design and consists of a first part (36a) connected to the pull cable (10) and a second part (36b) mounted on the non-rotatable shaft (2), wherein both parts (36a and 36b) of the anti-rotation device (36) stand in an operative connection with each other such that the second part (36b) blocks a rotation of the first part (36a) relative to the second part (36b).

3. Medical instrument according to claim 2, **characterized in that** the first part (36a) of the anti-rotation device (36) connected to the pull cable (10) is fashioned as a tube (28) coaxially enclosing the pul cable (10) and the second part (36b) of the anti-rotation device (36) mounted on the shaft (2) is fashioned as a sleeve (37) at least partially grasping the tube (28).

4. Medical instrument according to claim 2 or 3, **characterized in that** the two parts (36a, 36b) of the anti-rotation device (36) can be moved relative to each other in the direction of the lengthwise axis (14) of the shaft (2).

5. Medical instrument according to claims 3 and 4, **characterized in that** the tube (28) forming the first part (36a) of the anti-rotation device (36) has an outer contour which is not round in cross section, and the sleeve (37) forming the second part (36b) of the anti-rotation device (36) has an inner surface contour in the region grasping the tube (28) forming a corresponding mating contour to the outer contour of the tube (28) in this grasped region.

6. Medical instrument according to claim 5, **characterized in that** the tube (28) forming the first part (36a) of the anti-rotation device (36) has two flats (28b) on its outer envelope surface running in the direction of the lengthwise axis (14) of the shaft (2) and the sleeve (37) forming the second part (36b) of the anti-rotation device (36) has two corresponding flats (37a) on the inner side facing the tube (28) in the region grasping the tube (28).

7. Medical instrument according to claim 6, **characterized in that** the flats (28b) formed on the outer envelope surface of the tube (28) are staggered with respect to each other by 180°.

8. Medical instrument according to claims 3 and 4, **characterized in that** an oblong hole (28c) running in the direction of the lengthwise axis (14) of the shaft (2) is formed in the tube (28) forming the first part (36a) of the anti-rotation device (36) and a pin (37b) running radially and protruding into the oblong hole (28c) of the tube (28) is arranged in the sleeve (37) forming the second part (36b) of the anti-rotation device (36).

## Revendications

1. Instrument médical ayant une tige (2) creuse, à l'extrémité proximale (3) de laquelle est disposée une poignée (4) et à l'extrémité distale (5) de laquelle est disposée un embout d'outil (6), ledit embout d'outil (6) comportant un outil (7) pouvant être actionné au moyen d'un câble de traction (10) contraint par ressort et ledit embout d'outil (6) étant monté de manière rotative autour de l'axe longitudinal (14) de la tige (2) et de manière orientable sur la tige (2) par rapport à l'axe longitudinal (14) de la tige (2), l'actionnement du câble de traction (10) ainsi que la rotation et l'orientation de l'embout d'outil (6) étant obtenus au moyen d'entraînements (11, 15, 16) séparés respectifs disposés sur la poignée (4),
**caractérisé en ce qu'**
à l'extrémité distale du câble de traction (10) disposée dans l'embout d'outil (6) rotatif autour de l'axe longitudinal (14) de la tige (2), est disposé un blocage de rotation (36) protégeant le câble de traction (10) contre une torsion, ledit blocage de rotation (36) étant au moins partiellement monté sur la tige (2) non rotative.

2. Instrument médical selon la revendication 1, **caractérisé en ce que** le blocage de rotation (36) est structuré en deux parties et consiste en une première partie (36a) reliée au câble de traction (10) et en une deuxième partie (36b) montée sur la tige (2) non rotative, les deux parties (36a et 36b) du blocage de rotation (36) coopérant l'une avec l'autre de telle manière que la deuxième partie (36b) bloque une rotation de la première partie (36a) par rapport à la deuxième partie (36b).

3. Instrument médical selon la revendication 2, **caractérisé en ce que** la première partie (36a) du blocage de rotation (36) reliée au câble de traction (10) est réalisée comme tube (28) entourant coaxialement le câble de traction (10), et **en ce que** la deuxième partie (36b) du blocage de rotation (36) montée sur la tige (2) est réalisée comme douille (37) enserrant au moins partiellement le tube (28).

4. Instrument médical selon la revendication 2 ou 3, **caractérisé en ce que** les deux parties (36a, 36b) du blocage de rotation (36) sont mobiles l'une par rapport à l'autre dans le sens de l'axe longitudinal (14) de la tige (2).

5. Instrument médical selon les revendications 3 et 4, **caractérisé en ce que** le tube (28) formant la première partie (36a) du blocage de rotation (36) a un contour extérieur non circulaire en section transversale, et **en ce que** la douille (37) formant la deuxième partie (36b) du blocage de rotation (36) a un contour de surface intérieure dans la zone enserrant le tube (28), lequel forme un contour complémentaire correspondant au contour extérieur du tube (28) dans ladite zone enserrée.

6. Instrument médical selon la revendication 5, **caractérisé en ce que** le tube (28) formant la première partie (36a) du blocage de rotation (36) présente sur sa surface périphérique extérieure deux méplats (28b) s'étendant dans le sens de l'axe longitudinal (14) de la tige (2), et **en ce que** deux méplats (37a) correspondants sont formés sur la douille (37) formant la deuxième partie (36b) du blocage de rotation (36) dans la zone enserrant le tube (28), sur la face intérieure opposée au tube (28).

7. Instrument médical selon la revendication 6, **caractérisé en ce que** les méplats (28b) formés sur la surface périphérique extérieure du tube (28) sont décalés de 180° l'un par rapport à l'autre.

8. Instrument médical selon les revendications 3 et 4, **caractérisé en ce que** dans le tube (28) formant la première partie (36a) du blocage de rotation (36) est ménagé un trou oblong (28c) s'étendant dans le sens de l'axe longitudinal (14) de la tige (2), et **en ce que** dans la douille (37) formant la deuxième partie (36b) du blocage de rotation (36) est disposée une goupille (37b) s'étendant radialement et s'engageant dans le trou oblong (28c) du tube (28).
